(19) Europäisches Patentamt
European Patent Office
Office européen des brevets

(11) Veröffentlichungsnummer: **0 128 272**
**B1**

(12) # EUROPÄISCHE PATENTSCHRIFT

(45) Veröffentlichungstag der Patentschrift: **15.10.86**

(51) Int. Cl.⁴: **C 07 C 143/15**, A 61 K 7/13

(21) Anmeldenummer: **84102674.3**

(22) Anmeldetag: **12.03.84**

(54) **2,4-Diaminobenzolderivate, ihre Herstellung und Verwendung als Kuppler für Oxidationshaarfärbemittel.**

(30) Priorität: **19.03.83 DE 3309913**

(43) Veröffentlichungstag der Anmeldung: **19.12.84 Patentblatt 84/51**

(45) Bekanntmachung des Hinweises auf die Patenterteilung: **15.10.86 Patentblatt 86/42**

(84) Benannte Vertragsstaaten: **DE**

(56) Entgegenhaltungen:
**EP-A-0 039 455**
**CH-A-590 203**
**FR-A-1 558 842**
**US-A-2 740 808**
**US-A-2 913 451**

**Die Akte enthält technische Angaben, die nach dem Eingang der Anmeldung eingereicht wurden und die nicht in dieser Patentschrift enthalten sind.**

(73) Patentinhaber: **Henkel Kommanditgesellschaft auf Aktien, Postfach 1100 Henkelstrasse 67, D-4000 Düsseldorf- Holthausen (DE)**

(72) Erfinder: **Konrad, Günther, Dr., Feuerbachweg 12, D-4010 Hilden (DE)**
Erfinder: **Lieske, Edgar, Hunsrückenstrasse 40, D-4000 Düsseldorf (DE)**
Erfinder: **Rose, David, Dr., Holbeinweg 7, D-4010 Hilden (DE)**

**EP 0 128 272 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Gegenstand der Erfindung sind neue 2,4-Diaminophenylalkansulfonsäuren und 2,4-Diaminophenoxyalkansulfonsäuren und deren Alkali- und Ammoniumsalze. Diese Verbindungen sind wertvolle Vorprodukte für Oxidationshaarfärbemittel.

Für das Färben von Haaren spielen die sogenannten Oxidationsfarben, die durch oxidative Kupplung einer oder mehrerer Entwicklerkomponenten untereinander oder mit einer oder mehreren Kupplerkomponenten entstehen, wegen inhrer intensiven Farben und guten Echtheitseigenschaften eine bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopyridinderivate, heterocyclische Hydrazonderivate, 4-Aminopyrazolonderivate und Tetraaminopyrimidine eingesetzt. Als sogenannte Kupplersubstanzen werden m-Phenylendiaminderivate, Phenole, Naphthole, Resorcinderivate und Pyrazolone verwendet.

Gute Oxidationshaarfarbstoffvorprodukte müssen in erster Linie folgende Voraussetzungen erfüllen: Sie müssen bei der oxidativen Kupplung die gewünschten Farbnuancen in ausreichender Intensität ausbilden. Diese Farbnuancen müssen gegen die Einwirkung von Wärme, Licht und Chemikalien, z.B. gegen die bei der Kaltwellbehandlung des Haares verwendeten Reduktionsmittel eine ausreichende Stabilität aufweisen. Sie müssen ferner eine gutes Aufziehvermögen auf menschlichem Haar bestizen ohne die Kopfhaut zu stark anzufärben, und sie sollen vor allem in toxikologischer und dermatologischer Hinsicht unbedenklich sein.

Diese Anforderungen werden von den zur Zeit in Oxidationshaarfärbemitteln gebräuchlichen Kupplern, insbesondere von Kupplern, die mit bekannten Entwicklern wie p-Toluylendiamin blaue Nuancen ergeben, nicht zufriedenstellend erfüllt. Besonders problematisch sind die toxikologischen Eigenschaften vieler Kuppler z.B. vom Typ der aromatischen Amine. Andere Kupplersubstanzen führen zu Färbungen mit nicht befriedigenden Echtheitseigenschaften.

2,4-Diaminobenzolderivate sind als Kuppler für Oxidationsfärbemittel schon lange bekannt. Die Verwendung von 2,4-Diaminoanisol wird z.B. schon in DE—PS 226 790 und in DE—PS 228 245 für die Herstellung von Oxidationsfärbemitteln beschrieben. Zahlreiche Verbindungen dieser Stoffklasse weisen jedoch ungünstige toxikologische Eigenschaften, z.B. sensibilisierende oder mutagene Eigenschaften auf.

2,4-Diaminophenylalkancarbonsäuren und 2,4-Diaminophenoxyalkancarbonsaüren werden in DE—OS 30 16 881 als Kupplerkomponenten für Oxidationshaarfarben beschrieben. Auch diese Kupplersubstanzen befriedigen noch nich im Hinblick auf ihre toxikologischen Eigenschaften.

Bei der Suche nach besseren Oxidationsfarbstoffvorprodukten bestand daher die Aufgabe, geeignete Kupplersubstanzen aufzufinden, die einerseits mit bekannten Entwicklern brillante Färbungen von hoher Echtheit erzeugen, andererseits aber gegenüber den bekannten Kupplersubstanzen eine verringerte Toxizität aufweisen.

Es wurde nun überraschend gefunden, daß 2,4-Diaminobenzolderivate der allgemeinen Formel I

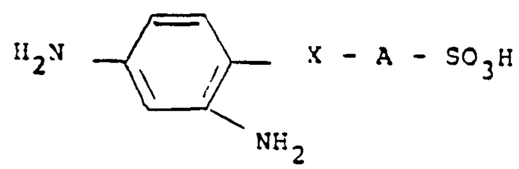

I

in der X ein Sauerstoffatom bedeutet oder nicht vorhanden ist und A eine Alkylengruppe mit 1—4 C-Atomen darstellt sowie deren Alkali- und Ammoniumsalze, den gestellten Anforderungen in hohem Maße gerecht werden. Diese Verbindungen sind neu und stellen ausgezeichnete Kupplersubstanzen für die Herstellung von Oxidationshaarfärbemitteln dar.

Die Herstellung der neuen Kupplersubstanzen der allgemeinen Formel I erfolgt durch katalytische Hydrierung der entsprechenden 2,4-Dinitrobenzolderivate der allgemeinen Formel II

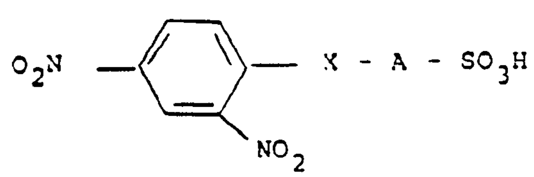

II

in der X und A die für die allgemeine Formel I angegebene Bedeutung haben, oder deren Alkali- oder Ammoniumsalze in ansich bekannter Weise.

Die 2,4-Dinitrobenzolderivate der allgemeinen Formel II lassen sich nach bekannten Syntheseverfahren herstellen. Dabei hat sich für die Verbindungen der allgemeinen Formel II, in welchen X nicht vorhanden ist, z.B. der folgende Syntheseweg bewährt:

Das entsprechende 2,4-Dinitrophenyl-alkylhalogenid der allgemeinen Formel III

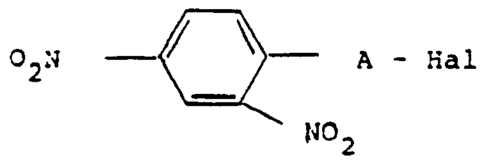

III

in der A eine Alkylengruppe mit 1—4 C-Atomen und Hal ein Halogen, z.B. Chlor oder Brom bedeutet, wird mit Alkalisulfit, z.B. $Na_2SO_3$, in das Alkalisalz der entsprechenden Verbindung der allgemeinen Formel II überführt.

Für Verbindungen der allgemeinen Formel II, in welchen X ein Sauerstoffatom bedeutet, hat sich besser die Nitrierung der entsprechenden-ω-Phenoxyalkansulfonsäure der allgemeinen Formel IV bewährt,

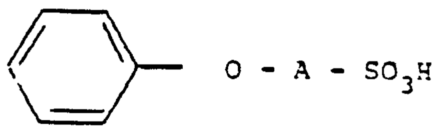

IV

in der A die für die Formel I angegebene Bedeutung hat. Diese Nitrierung läßt sich z.B. ohne Schwierigkeiten durch Eintragen eines Alkalisalzes der entsprechenden ω-Phenoxyalkansulfonsäure in konzentrierte Salpetersäure und Umsetzung unter Kühlung bei Temperaturen unterhalb +20°C durchführen.

Geignete neue Kupplersubstanzen der allgemeinen Formel I sind z.B.
2,4-Diaminophenyl-methansulfonsäure,
β-(2,4-Diaminophenyl)-ethansulfonsäure,
β-(2,4-Diaminophenyl)-propansulfonsaüre,
γ-(2,4-Diaminophenyl)-propansulfonsaüre,
β-(2,4-Diaminophenyl)-butansulfonsäure,
γ-(2,4-Diaminophenyl)-butansulfonsäure,
δ-(2,4-Diaminophenyl)-butansulfonsäure,
β-(2,4-Diaminophenooxy)-ethansulfonsäure,
β-(2,4-Diaminophenoxy)-propansulfonsäure,
γ-(2,4-Diaminophenoxy)-propansulfonsäure,
γ-(2,4-Diaminophenoxy)-butansulfonsäure,
oder deren Alkali- oder Ammoniumsalze.

Bevorzugt werden die erfindungsgemäßen Kuppler in Form der Alkalisalze, insbesondere als Natrium- oder Kaliumsalze verwendet. In den Verbindunger der allgemeinen Formel I, in welchen X nicht vorhanden ist, stellt A bevorzugt eine $—(CH_{2n}—$ Gruppe dar, in welcher n = 1—3 ist. Von den Verbindungen der allgemeinen Formel I, in welchen X ein Sauerstoffatom bedeutet, stellt A bevorzugt eine $—(CH_2)_n—$Gruppe dar, in welcher n = 2—4 ist. Besonders bevorzugte Verbindungen der allgemeinen Formel I sind daher die Natrium- oder Kaliumsalze von
2,4-Diaminophenyl-methansulfonsäure
β-(2,4-Diaminophenyl)-ethansulfonsäure
γ-(2,4-Diaminophenyl)-propansulfonsäure
β-(2,4-Diamonophenoxy)-ethansulfonsäure
γ-(2,4-Diaminophenoxy)-propansulfonsäure
γ-(2,4-Diaminophenoxy)-butansulfonsäure
oder deren Alkali- oder Ammoniumsalze.

Die erfindungsgemäßen neuen Kupplersubstanzen eignen sich für eine Vielzahl verschiedener Entwicklersysteme. Sie erzeugen auf dem Haar intensive, tiefblaue bis braune Farbnuancen vonn hoher Reinheit und guter Beständigkeit gegen Licht und Wärme. Sie haben darüber hinaus besonders günstige toxikologische Eigenschaften, die sie für die Verwendung in kosmetischen Haarfärbemitteln besonders geeignet machen. Die neuen Kupplersubstanzen der allgemeinen Formel I stellen daher eine wertvolle Bereichrung der oxidativen Haarfärbemöglichkeiten dar.

Besonders intensive und reine blaue Farbnuancen werden bei Verwendung der neuen Kupplersubstanzen in Oxidationsfärbemitteln zusammen mit Entwicklersubstanzen vom Typ der aromatischen Diamine, insbesondere der p-Phenylendiamin-Derivate erhalten. Solche blauen Nuancen sind für die Erzeugung von Brauntönen durch Kombination mit gelben Nuancen sowie zur Kompensation von Farbverschiebungen in Blondiermitteln von besonderem anwendungstechnischem Wert.

Solche bevorzugten Entwicklersubstanzen sind z.B. p-Phenyldiamin, p-Toluylendiamin, N-Methyl-p-phenylendiamin, N,N-Dimethyl-p-phenylendiamin, N,N-Diethyl-2-methyl-p-phenylendiamin, N-Ethyl-N-hydroxyethyl-p-phenylendiamin, N-Hydroxyethyl-p-phenylendiamin, Chlor-p-phenylendiamin, N,N-Bis-hydroxyethylamino-p-phenylendiamin, Methoxy-p-phenylendiamin, 2,6-Dichlor-p-phenylendiamin, 2-Chlor-6-brom-p-phenylendiamin, 2-Chlor-6-methyl-p-phenylendiamin, 6-Methoxy-3-methyl-p-phenylendiamin, 2,5-Diaminoanisol, N-Ethyl-(N-(2-hydroxyethyl)-p-phenylendiamin, N-2-Methyoxyethyl-p-phenylendiamin, N-2-Hydroxypropyl-p-phenylendiamin, N-Butyl-N-sulfobutyl-p-phenylendiamin oder deren Salze mit anorganischen oder organischen Säuren.

Ein weiterer Erfindungsgegenstand sind Haarfärbemittel, enthaltend Oxidationsfarbstofvorprodukte in einem kosmetischen Träger, dadurch gekennzeichnet, daß als Oxidationsfarbstoffvorprodukte Kupplersubstanzen der allgemeinen Formel I neben üblichen Kupplersubstanzen und üblichen Entwicklersubstanzen enthalten sind. Als Entwicklersubstanzen sollen bevorzugt die genannten aromatischen Diamine, insbesondere p-Phenylendiamin-Derivate oder deren Salze, enthalten sein.

Daneben können die erfindungsgemäßen Haarfärbemittel jedoch zur Erzeugung der gewünschten Farbtöne noch andere bekannte Entwickler sowie Kupplerkomponenten enthalten. Solche Entwickler sind z.B. p-Aminophenole, Diaminopyridin-Derivate, heterocyclische Hydrazonderivate, 4-Aminopyrazolonderivate und Tetraaminopyrimidine. Als weitere Kuppler können den erfindungsgemäßen Haarfärbemitteln z.B. Naphthole, Resorcinderivate, Pyrazolone und m-Phenylendiaminderivate zugesetzt werden. Auch direktziehende Farbstoffe, z.B. Nitrophenylendiaminderivate können zu Modifikation der Farbnuancen zugesetzt werden.

Die erfindungsgemäß zu verwendenden Kupplersubstanzen der allgemeinen Formel I sind in solchen Haarfärbemitteln je nach der gewünschten Nuance in Mengen von 0,01—3,0 Gew.-%, bezogen auf das gesamte Mittel, enthalten. In den erfindungsmäßen Haarfärbemitteln werden die Kupplersubstanzen im allgemeinen in etwa molaren Mengen, bezogen auf die verwendeten Entwicklersubstanzen, eingesetzt. Wenn sich auch der molare Einsatz als zweckmässig erweist, so ist es jedoch nicht nachteilig, einen gewissen Überschuß einzelner Oxidationsfarbstoffvorprodukte zum Einsatz zu bringen.

Es ist ferner nich erforderlich, daß die Oxidationsfarbstoffvorprodukte einheitliche Substanzen darstellen, vielmehr können sowohl die Entwicklerkomponente Gemische der erfindungsgemäß zu verwendenden Entwicklerverbindungen als auch die Kupplerkomponente Gemische der erfindungsgemäß einzusetzenden 2,4-Diaminobenzolderivate der allgemeinen Formel I und bekannter Kupplersubstanzen darstellen.

Die oxidative Entwicklung der Färbung kann grundsätzlich, wie bei anderen Oxidationshaarfarbstoffen auch durch Luftsauerstoff erfolgen. Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Zur Herstellung der erfindungsgemäßen Haarfärbemittel werden die Oxidationsfarbstoff-Vorprodukte in einen geeigneten kosmetischen Träger eingearbeitet. Solche Träger sind z.B. Cremes, Emulsionen, Gele oder auch tensidhaltige, schäumende Lösungen, z.B. Shampoos oder andere Zubereitungen, die für die Anwendung auf dem Haar geeignet sind. Übliche Bestandteile solcher kosmetischer Zubereitungen sind zum Beispiel Netz- und Emulgiermittel, wie anionsche, nichtionische oder ampholytische Tenside, z.B. Fettalkoholsulfate, Alkansulfonate, α-Olefinsulfonate, Fettalkoholpolyglykoethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel wie z.B. Methyl- oder Hydroxyethylcellulose, Stärke, Fettalkohole, Paraffinöl, Fettsäuren, ferner Parfümöle udn haarpflegende Zusätze wie z.B. wasserlösliche kationische Polymere, Proteinderivate, Pantothensäure und Cholesterin.

Die Bestandteile der kosmetischen Träger werden zu Herstellung der erfindungsgemäßen Haarfärbemittel in für diese Zwecke üblichen Mengen eingesetzt; z.B. werden Emulgiermittel in Konzentrationen von 0,5 bis 30 Gew.-% und Verdickungsmittel in Konzentrationen von 0,1 bis 25 Gew.-% der Haarfärbemittel eingesetzt. Die Oxidationsfarbstoffvorprodukte werden üblicherweise in Mengen von 0,05 bis 5,0 Gew.-% des gesamten Färbemittels in den Träger eingemischt.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8—10 erfolgen. Die Anwendungstemperaturen bewegen sich dabei im Bereich von 15 bis 40°C. Nach einer Einwirkungsdauer von ca. 30 Minuten wird das Haarfärbemittel vom zu färbenden Haar durch Spülen entfernt. Hernach wird das Haar mit einem milden Shampoo nachgewaschen und getrocknet. Das Nachwaschen mit einem Shampoo entfällt, ween ein stark tensidhaltiger Träger, z.B. ein Färbeshampoo, verwendet wurde.

Die mit den erfindungsgemäßen Haarfärbemitteln erzielbaren Färbungen haben gute Licht-, Wasch- und Reibechtheitseigenschaften.

Die nachfolgenden Beispiele sollen den Erfindungsgegenstand näher erläutern, ohne ihn jedoch hierauf zu beschränken.

## Beispiele

### 1. 2,4-Diaminophenyl-methansulfonsäure-Natriumsalz

a) 2,4-Dinitrophenyl-methanfulfonsäure, Na-Salz

20 g 2,4-Dinitrobenzylchlorid*) (0,092 Mol) wurden in 50 ml Ethanol gelöst. Zu dieser Lösung wurde eine Lösung von 11,6 Natriumsulfit, wasserfrei (0,092 Mol) in 40 ml Wasser bei Raumtemperatur (20°C) zugegeben. Die Temperature der Reaktionnsmischung stieg dabei auf 32°C an. Dann wurde die Reaktionsmischung 5 Stunden unter Rückfluß zum Sieden erhitzt. Nach dem Abkühlen auf

Raumtemperatur wurde das ausgefallene, orangefarbene Produkt durch Filtration abgetrennt und mit 20 ml Ethanol gewaschen.

Zur Reinigung wurde das Rohprodukt mit 500 ml Aceton verrührt. Der darin nicht gelöste Rückstand wurde abgetrennt und in Ethanol gelöst. Die Lösung wurde filtriert und das Filtrat durch Destillation eingeengt. Das ausgefallene Produkt wurde abfiltriert und bei 70°C unter vermindertem Druck getrocknet. Auf diese Weise wurde eine Ausbeute von 12 g erhalten.

Analyse:
Berechnet für $C_7H_5N_2O_7SNa + 0{,}2\ NaCl + 0{,}5\ H_2O$

| | | | | |
|---|---|---|---|---|
| theor.: | C: 27,57 | H: 1,97 | N: 9,19 | Cl: 2,33 |
| gefunden: | C: 27,8 | H :2,23 | N: 8,93 | Cl: 2,55 |

*) 2,4-Dinitrobenzylchloride ist ein Handelsprodukt der Fa. Merck, Darmstadt

b) 2,4-Diaminophenyl-methansulfonsäure, Na-Salz

5,5 g des Produktes gemäß Beispiel 1a) wurden in 200 ml Wasser suspendiert und in Gegenwart von Palladium-Katalysator (5 Gew.-% Pd auf Aktivkohle) hydriert. Es wurden 2,75 l Wasserstoff (berechnet 2,70 l $h_2$) aufgenommen. Nach Beendigung der Wasserstoff-Aufnahme wurde der Katalysator abfiltriert und das Filtrat zur Trockene eingedampft. Das Produkt wurde als Rückstand in einer Menge von 3,8 g erhalten.

Analyse:
Berechnet für $C_7H_9H_2O_3SNa + 0{,}2\ NaCl$

| | | | | |
|---|---|---|---|---|
| theor.: | C: 35,52 | H: 3,83 | N: 11,83 | S: 13,54 |
| gefunden: | C: 35,4 | H: 3,78 | N: 12,1 | S: 13,4 |

$^1$H—NMR-Spektrum in $d_6$-DMSO mit H—D-Austausch (δ in ppm) (gemessen bei 60 MHz)
δ = 6,0—6,6 (m, 3H); 3,7 (s, 2H);

2. β-(2,4-Diaminophenyl)-enthansulfonsäure-Natriumsalz

a) β-(2,4-Dinitrophenyl)-ethansulfonsäure, Na-Salz

Zu 6,3 g β-(2,4-Dinitrophenyl)-ethylbromid*), (0,023 Mol), gelöst in 10 ml Ethanol, wurde unter Rühren eine Lösung von 2,9 g (0,023 Mol) Natriumsulfit, wasserfrei (0,023 Mol) in 5 ml Wasser gegeben. Die Temperatur der Mischung stieg dabei von 20°C auf 45°C. Dann wurde die Reaktionsmischung 5 Stunden unter Rückfluß zum Sieden erhitzt. Nach dem Abkühlen auf Raumtemperatur wurde das ausgefallene, gelbe Produkt durch Filtration abgetrennt, in 10 ml Wasser gelöst, von wenig ungelöstem Rückstand abfiltriert und auf 15 ml eingeengt. Nach dem Abfiltrieren des ausgefallenen Produktes wurde dieses bei 80°C unter vermindertem Druck getrocknet. Auf diese Weise wurde eine Ausbeute von 1,9 g erhalten.

Analyse:
Berechnet für $C_8H_7N_2O_7SNa$

| | | | | | |
|---|---|---|---|---|---|
| theor.: | C: 30,38 | H: 2,85 | N: 8,86 | S: 10,13 | Na: 7,28 |
| gefunden: | C: 29,9 | H: 2,70 | N: 8,43 | S: 10,2 | Na: 7,18 |

*) herstellbar z.B. nach K. Issleib et al, Z. anorg. allg. Chemie, Bad. 424, (1976), S. 101

b) β-(2,4-Diaminophenyl)-ethansulfonsäure, Na-Salz

1,5 g des Produktes gemäß Beispiel 2a) wurden in 50 ml Wasser gelöst und in Gegenwart von Palladium-Katalysator (5 Gew.-% Pd auf Aktivkohle) hydriert. Nach beendeter Hydrierung wurde der Katalysator abfiltriert und das Filtrat zur Trockene eingedampft. Das Produkt wurde als Rückstand in einer Menge von 0,9 g erhalten.

Analyse:
Berechnet für $C_8H_{11}N_2O_3SNa + H_2O$

| | | | | | |
|---|---|---|---|---|---|
| theor.: | C: 37,5 | H: 5,08 | N: 10,94 | S: 12,5 | Na: 8,98 |
| gefunden | C: 36,3 | H: 4,94 | N: 10,2 | S: 11,9 | N: 8,75 |

3. β-(2.4-Diaminophenoxy)-ethansulfonsäure-Natriumsalz

a) β-(2,4-Dinitrophenoxy-ethansulfonsäure, Na-Salz

40 g 2-Phenoxy-ethansulfonsäure, Na-Salz*) (0,178 Mol) wurden bei 15—20°C unter Wasserkühlung portionsweise zu 132 ml konzentrierter Salpetersäure (Dichte: 1,52) unter Rühren zugegeben. nach 15

Minuten wurden 100 ml Wasser zugesetzt. Dann wurde das Reaktionsgemisch bei 40—50°C unter vermindertem Druck (Wasserstrahlvakuum) eingedampft. Der Rückstand wurde dreimal mit je 100 ml Wasser aufgenommen und erneut eingedampft. Auf diese Weise wurden 54 g Rohproodukt erhalten. Durch Umkreistallisation aus Wasser wurden 34 g eines bei 168—172°C schmelzenden Produktes erhalten

Analyse:
Berechnet für $C_8H_7O_8SN_2Na + 0{,}5H_2O$

| | | | | | |
|---|---|---|---|---|---|
| theor.: | C: 29,73 | H: 2,49 | N: 8,67 | S :9,92 | Na: 7,11 |
| gefunden: | C: 29,5 | H: 2,31 | N: 8,58 | S: 9,61 | Na: 6,95 |

---

*) herstellbar aus β-Bromophenetol und Natriumsulfit analog der Umsetzung von 2-Phenylethylbromid mit Natriumsulfit nach E. B. Evans et al, J. chem Soc, 1927, S 1161.

b) β-(2,4-Diaminophenoxy)-ethansulfonsäure, Na-Salz

10 g des Produktes gemäß Beispiel 3a wurden in 200 ml Wasser gelöst und in Gegenwart von Palladium-Katalysator (5 Gew.-% Pd auf Aktivkohle) hydriert. Nach 2,5 Stunden war die Wasserstoffaufnahme beendet. Nach Abtrennung des Katalysators wurde das Filtrat zur Trockene eingedampft. Es verblieben 7,5 g eines bei 126°—128°C schmelzenden Produktes als Rückstand.

Analyse:
Berechnet für $C_8H_{11}N_2O_4SNa + H_2O$

| | | | | |
|---|---|---|---|---|
| theor.: | C: 35,29 | H: 4,81 | N: 10,29 | S: 11,78 |
| gefunden | C: 34,9 | H: 4,59 | N: 10,1 | S: 11,6 |

$^1$H—NMR-Spektrum in $d_6$-DMSO mit H—D—Austausch (δ in ppm) (gemessen bei 60 MHz)
γ = 6,1—6,7 (m, 3H); 4,2—4,4 (t, 2H); 3,2—3,5 (t, 2H);

4. δ-(2,4-Diaminophenoxy)-prpansulfonsäure, Kalimsalz

a) γ-(2,4-Dinitrophenoxy)-propansulfonsäure, K-Salz

10 g γ-Phenoxypropansulfonsäure, K-Salz*) (0,039 Mol) wurden bei −5°C portionwise zu 30 ml konzentrierter Salpetersäure (Dichte: 1,52) gegeben. Nach 15 Minuten Nachrühren be −5°C wurde das Reaktionsgemisch auf Eis gegossen und dann unter vermindertem Druck bei 40—50°C eingedampft. Der Rückstand wurde aus 10 ml Wasser umkristallisiert und ergab 1,9 g Ausbeute.

Anaylyse:
Berechnet für $C_9H_9N_2O_8SK + H_2O$

| | | | |
|---|---|---|---|
| theor.: | C: 29,83 | H: 3,06 | N: 7,73 |
| gefunden | C: 28,6 | H: 2,54 | N: 7,99 |

$^1$H—NMR-Spektrum in $d_6$—DMSO (δ in ppm) δ = 7,5—8,8 (m, 3H); 4,3—4,7 (t, 2H); 2,5—2,8 (t, Überlappung mit DMSO); 1,9—2,4 (m, 2H)

---

*) γ-Phenoxypropansulfonsäure, K-Salz ist ein Handelsprodukt der Fa. Raschig.

b) γ-(2,4-Diaminophenoxy)-propansulfonsäure, K-Salz

1 g des Produktes gemäß Beispeil 4a) wurde in 50 ml Wasser gelöst und in Gegenwart von Palladium-Katalyasator (5 Gew.-% Pd auf Aktivkohle) hydriert. Nach Beendigung der Wasserstoffaufnahme wurde die Lösung von Katalysator abfiltriert und durch Eindampfer unter vermindertem Druck zur Trockene eingeengt. Es wurden als Rückstand 0,8 g einer hydroskopischen Substanz isoliert.

Anwendungstechnische Prüfung

Zur Prüfung der neuen Kupplerkomponenten gemäß Beispiel 1—4 wurden diese in Haarfärbe-Cremeemulsionen der folgenden Zusammensetzung eingesetzt:

| | |
|---|---|
| Fettalkohol $C_{12-18}$ | 10 g |
| Fettalkohol $C_{12-14}$ + 2EO-sulfat, Na-Salz (28%ig) | 25 g |
| Wasser | 60 g |
| Entwicklersubstanz | 0,0075 Mol |
| Kupplersubstanz | 0,0075 Mol |

| | |
|---|---|
| Inibitor ($Na_2SO_3$) | 1,0 g |
| konz. Ammoniak-Lösung | bis pH = 9,5 |
| Wasser | ad 100 g |

Die Bestandteile wurden der Reihe nach miteinander vermischt. Nach Zugabe der Entwicklersubstanz und der Kupplersubstanz wurde zunächst mit konzentrierter Ammoniak-Lösung derpH-Wert der Emulsion auf 9,5 einge stellt, dann mit Wasser auf 100 g aufgefüllt.

Die oxidative Kupplung wurde mit 3%iger Wasserstoffperoxidlösung als Oxidationsmittel durchgeführt. Hierzu wurden 10 g der Fäbecreme mit 5 g Wasserstoffperoxidlösung (3%ig) versetzt und vermischt.

Die Färbecreme wurde auf standardisiertes, zu 90% ergrautes aber nicht besonders vorbehandeltes Menschenhaar aufgetragen und dort 30 Minuten bei 35°C belassen. Nach Beedingung des Färbeprozesses wurde das Haar gespült, mit einem üblichen Haarwaschmittel ausgewaschen und anschließend getrocknet.

Als Entwicklersubstanzen wurden die folgenden Verbindungen eingesetzt:

E 1: p-Toluylendiamin
E 2: N-Hydroxyethyl-p-phenylendiamin
E 3: 2-Chlor-p-phenylendiamin
E 4: 2,4,5,6,-Tetraaminopyrimidin
E 5: 2,5-Diaminopyridin

Als erfindungsgemäße Kupplersubstanzen wurden eingesetzt:

K 1: 2,4-Diaminophenyl-methansulfonsäure, Na-Salz
K 2: β-(2,4-Diaminophenyl)-ethansulfonsäure, Na-Salz
K 3: β-(2,4-Diaminophenoxy)-ethansulfonsäure, Na-Salz
K 4: γ-(2,4-Diaminophenoxy)-propansulfonsäure, K-Salz
Die Ergebnisse der Färbeveresuche sind in Tabelle I zusammengestellt.

TABELLE I

| Beispiel | Entwickler | Kuppler | Nuance des gefärbten Haares |
|---|---|---|---|
| P 1 | E 1 | K 1 | schwarzblau |
| P 2 | E 2 | K 1 | tinteblau |
| P 3 | E 3 | K 1 | dunkelviolett |
| P 4 | E 1 | K 2 | gewitterblau |
| P 5 | E 1 | K 3 | schwarzblau |
| P 6 | E 1 | K 4 | schwarzblau |
| P 7 | E 4 | K 1 | dunkelgrün |
| P 8 | E 4 | K 2 | khaki |
| P 9 | E 4 | K 4 | dunkelgrüp |
| P 10 | E 5 | K 1 | graubruan |

**Patentansprüche**

1. 2.4-Diaminobenzolderivate der allgemeinen Formel I

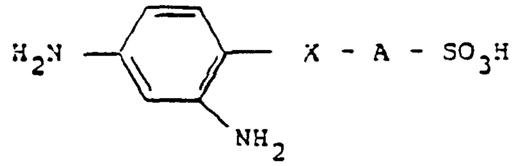

I

in der X ein Sauerstoffatom bedeutet oder nicht vorhanden ist un A eine Alkylengruppe mit 1—4 C-Atomen darstellt sowie deren Alkali- und Ammoniumsalze.

2. 2.4-Diaminobenzolderivate nach Anspruch 1, dadurch gekennzeichnet, daß X nicht vorhanden ist und A eine $-(CH_2)_n$-Gruppe darstellt, in der n = 1—3 ist.

3. 2.4-Diaminobenzolderivate nach Anspruch 1, dadurch gekennzeichnet, daß X ein Sauerstoffatom bedeutet und A eine $-(CH_2)_n$-Gruppe darstellt, in der n = 2—4 ist.

4. Verfahren zur Herstellung von Verbindungen der Formel I nach Anspruch 1, dadurch gekennzeichnet, daß man Verbindungen der allgemeinen Formel II

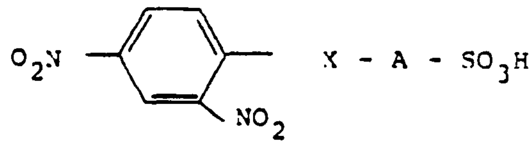

II

in der X und A die für die Formel I angegebene Bedeutung haben oder deren Alkali- oder Ammoniumsalze in an sich bekannter Weise katalytisch hydriert.

5. Verwendung der 2.4-Diaminobenzolderivate nach Anspruch 1—3 in Oxidationsfärbemitteln als Kupplersubstanzen zusammen mit üblichen Entwicklersubstanzen.

6 Verwendung nach Anspruch 5, dadurch gekennzeichnet, daß als Entwicklersubstanzen aromatische Diamine, insbesondere p-Phenylendiamin-Derivate eingesetzt werden.

7. Haarfärbemittel, enthaltend Oxidationsfarbstoffvorprodukte in einem kosmetischen Träger, dadurch gekennzeichnet, daß als Oxidationsfarbstoffvorprodukte Kupplersubstanzen der allgemeinen Formel I nach Anspurch 1 in einer Menge von 0,01—3,0 Gew.-%, bezogen auf des gesamte Mittel, neben üblichen Kupplersubstanzen und üblichen Entwicklersubstanzen enthalten sind.

8. Haarfärbemittle nach Anspurch 7, dadurch gekennzeichnet, daß als Entwicklersubstanzen aromatische Diamine, insbesondere p-Phenylendiaminderivate enthalten sind.

**Revendications**

1. Dérivés de 2,4-diaminobenzène de formule générale I:

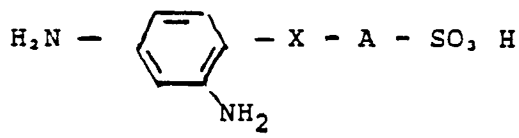

I

dans laquelle X signifie un atome d'oxygène ou n'est pas présent et A représente un groupe alcoylène ayant 1 à 4 atomes de carbone, de même que leurs sels alcalins et d'ammonium.

2. Dérivés de 2,4-diaminobenzène selon la revendication 1, caractérisés en ce que X n'est pas présent et A est un groupe $-(CH_2)_n-$ dans lequel n = 1—3.

3. Dérivés de 2,4-diaminobenzène selon la revendication 1, caractérisés en ce que X signifie un atome d'oxygène et A un groupe $-(CH_2)_n$ dans lequel n = 2—4.

4. Procédé de préparation de composés de formule I selon la revendication 1, caractérisé en ce qu'on hydrogène catalytiquement d'une manière connue en elle-même des composés de formule générale II:

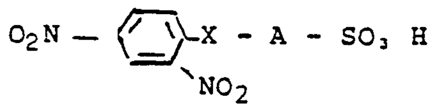

II

dans laquelle X et A ont la signification indiquée à la formule I, ou leurs sels alcalins ou d'ammonium.

5. Utilisation des dérivés de 2,4-diaminobenzène selon les revendications 1 à 3 dans des colorants d'oxydation comme substances de couplage conjointement avec les substances de développement en usage.

6. Utilisation selon la revendication 5, caractérisé en ce qu'on utilise comme substances de développement des diamines aromatiques, en particulier des dérivés de p-phénylène diamine.

7. Agents de teinture pour cheveux, contenant des précurseurs de colorants d'oxydation dans un support cosmétique, caractérisé en ce qu'ils contiennent comme précurseurs de colorants d'oxydation des substances de couplage de formule générale I selon la revendication 1 en une quantité de 0,01 à 3,0% en poids par rapport à l'agent dans son ensemble, à côté des substances de couplage en usage et des substances de développement en usage.

8. Agents de teinture pour cheveux selon la revendication 7, caractérisé en ce qu'ils contiennent comme substances de développement des diamines aromatiques, en particulier des dérivés de p-phénylène diamine.

**Claims**

1. 2,4-diaminobenzene derivatives corresponding to the following general formula:

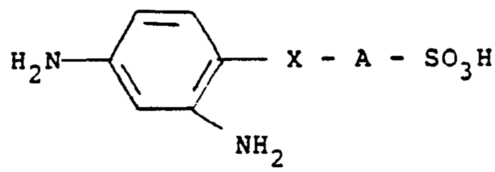

I

in which X is an oxygen atom or is not present and A is a $C_1$–$C_4$ alkylene group, and alkali and ammonium salts thereof.

2. 2,4-diaminobenzene derivatives as claimed in Claim 1, characterized in that X is not present as A is a $-(CH_2)_n-$ group where n = 1—3.

3. 2,4-diaminobenzene derivatives as claimed in Claim 1, characterized in that X is an oxygen atom and A is $-(CH_2)_n-$ group where n = 2—4.

4. A process for the preparation of compounds corresponding to formula I in Claim 1, characterized in that compounds corresponding to the following general formula

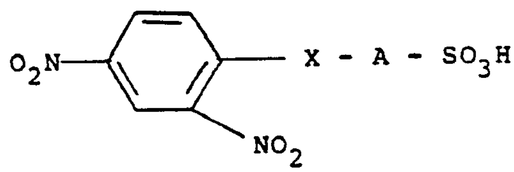

II

in which X and A are as defined for formula I, or alkali or ammonium salts thereof are catalytically hyrogenated by methods known per se.

5. The use of the 2,4-diaminobenzene derivatives claimed in Claims 1 to 3 in oxidation hair dyes as couplers togethere with standard developers.

6. The use claimed in Claim 5, characterized in that aromatic diamines, particularly p-phenylene diamine derivatives, are used as developers.

7. Hair dyes containing oxidation dye precursors in a cosmetic carrier, characterized in that they contain couplers corresponding to general formula I in Claim 1 in a quantity of from 0.01 to 3.0% by weight, based on the hair dye as a whole, as oxidation dye precursors in addition to standard couplers and standard developers.

8. Hair dyes as claimed in Claim 7, characterized in that aromatic diamines, particularly p-phenylene diamine derivatives, are present as developers.